(19) Europäisches Patentamt
European Patent Office
Office européen des brevets

(11) Numéro de publication: **0 320 368 B1**

# (12) FASCICULE DE BREVET EUROPEEN

(45) Date de publication de fascicule du brevet: **16.09.92**

(51) Int. Cl.[5]: **C07D 249/10**, C06B 25/04

(21) Numéro de dépôt: **88403087.5**

(22) Date de dépôt: **06.12.88**

## (54) 1-(3,5-Diamino-2,4,6-trinitrophényl)-3-nitro-1H-1,2,4-triazole, son procédé de préparation et matériau explosif le contenant.

(30) Priorité: **08.12.87 FR 8717058**

(43) Date de publication de la demande:
**14.06.89 Bulletin 89/24**

(45) Mention de la délivrance du brevet:
**16.09.92 Bulletin 92/38**

(84) Etats contractants désignés:
**CH DE GB IT LI SE**

(56) Documents cités:
**US-A- 2 987 520**
**US-A- 3 483 211**
**US-A- 3 715 398**

**JOURNAL OF HETEROCYCLIC CHEMISTRY, vol. 7, no. 6, décembre 1970, pages 1237-1239, Provoh, Utah, US; M.A. KHAN et al.: "Syntheses of N-(2,4-dinitrophenyl)nitroazoles"**

(73) Titulaire: **COMMISSARIAT A L'ENERGIE ATOMIOUE Etablissement de Caractère Scientifique Technique et Industriel**
**31/33, rue de la Fédération**
**F-75015 Paris(FR)**

(72) Inventeur: **Laval, François**
**Avenue Joliot Curie**
**F-37260 Monts(FR)**
Inventeur: **Vignane, Pascal**
**4 rue du Grand Marché**
**F-37000 Tours(FR)**

(74) Mandataire: **Mongrédien, André et al**
**c/o BREVATOME 25, rue de Ponthieu**
**F-75008 Paris(FR)**

EP 0 320 368 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne un nouveau dérivé de triazole, son procédé de préparation et son utilisation comme explosif.

De façon plus précise, elle concerne un nouveau dérivé de triazole utilisable comme explosif secondaire, dans l'industrie aérospatiale ou pour l'équipement des missiles et armements modernes.

Pour ces applications, il est intéressant d'utiliser des explosifs ayant une sensibilité au choc aussi faible que possible, mais une puissance élevée, c'est-à-dire la capacité de délivrer une énergie très élevée. Ces deux propriétés sont difficiles à trouver simultanément dans un même explosif. Ainsi, le triaminotrinitrobenzène (TATB) est très peu sensible au choc, mais il manque de puissance, alors que la cyclotétraméthylène-tétranitramine(octogène) qui est très puissante, est plus sensible au choc et aux agressions.

Aussi, des recherches ont été effectuées récemment pour mettre au point de nouveaux explosifs dont la sensibilité au choc se rapproche de celle du TATB tout en étant capables de délivrer une énergie plus élevée que ce dernier, se rapprochant de celle de l'octogène.

La présente invention a précisément pour objet un nouveau dérivé de triazole présentant ces propriétés.

Le nouveau dérivé de triazole de l'invention est le 5-nitro-2(3,5-diamino-2,4,6-trinitrophényl)-1,2,4-triazole répondant à la formule :

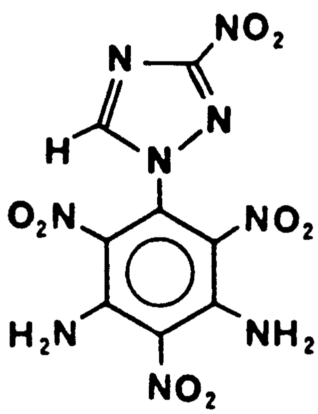

(I)

Ce nouveau dérivé de triazole présente un grand intérêt pour une utilisation comme explosif secondaire car ses propriétés détoniques sont intermédiaires entre celles du TATB et de l'octogène en ce qui concerne la sensibilité au choc et la vitesse de détonation.

Ce nouveau dérivé de triazole peut être préparé par un procédé consistant à faire réagir un 3,5-diamino-1-halogéno-2,4,6-trinitrobenzène de formule :

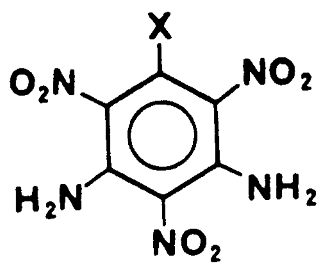

(II)

dans laquelle X représente un atome de chlore ou de fluor, avec un 3-nitro-1,2,4-triazole de formule :

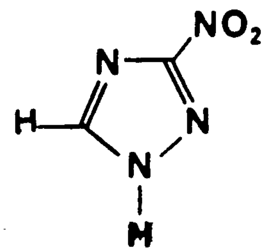

(III)

dans laquelle M représente un atome d'hydrogène ou de métal alcalin.

Cette réaction correspond au schéma réactionnel suivant :

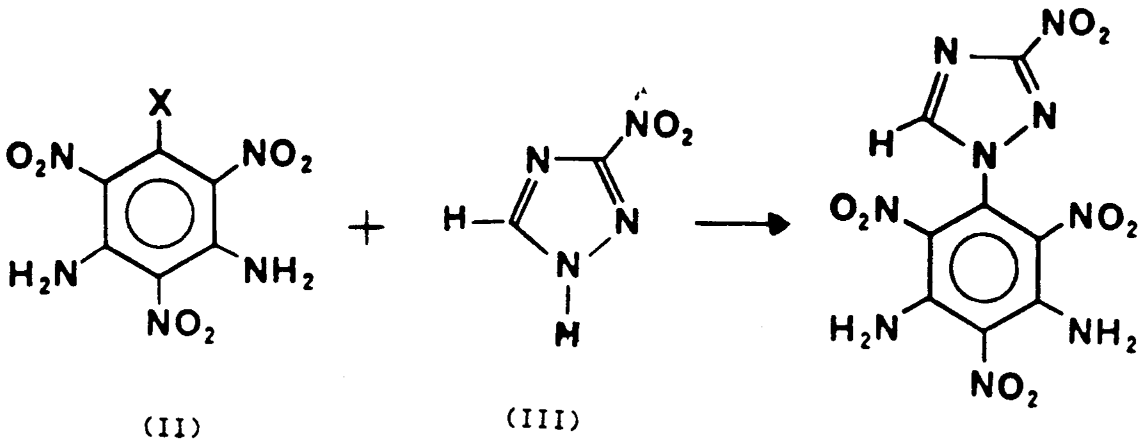

(II) (III) (I)

Lorsque X représente un atome de fluor, on peut réaliser la réaction à la température ambiante car l'atome de fluor présente une grande réactivité vis-à-vis des réactifs nucléophiles, en utilisant le triazole de formule III avec M représentant un atome d'hydrogène.

En revanche, lorsque X représente un atome de chlore, qui est moins réactif, M représente de préférence un atome de métal alcalin car il est préférable de faire réagir le 3,5-diamino-1-halogéno-2,4,6-trinitrobenzène avec un sel alcalin du 3-nitro-1,2,4-triazole. Il est également préférable de réaliser la réaction à une température supérieure à la température ambiante, par exemple à 50 ° C. Le sel alcalin peut être obtenu en faisant réagir le 3-nitro-1,2,4-triazole avec un alcoolate de métal alcalin, en particulier un alcoolate de lithium, de sodium, ou de potassium.

Les réactifs de départ utilisés pour la préparation de ce nouveau dérivé de triazole sont des produits du commerce ou peuvent être obtenus par des procédés classiques.

Ainsi, le 3,5-diamino-1-fluoro-2,4,6-trinitrobenzène peut être préparé par réaction du trifluorotrinitrobenzène avec une amine primaire très encombrée telle que la tert-butylamine, en éliminant ensuite les groupements tert-butyle par action de l'acide trifluoracétique. Cette synthèse correspond au schéma réactionnel suivant :

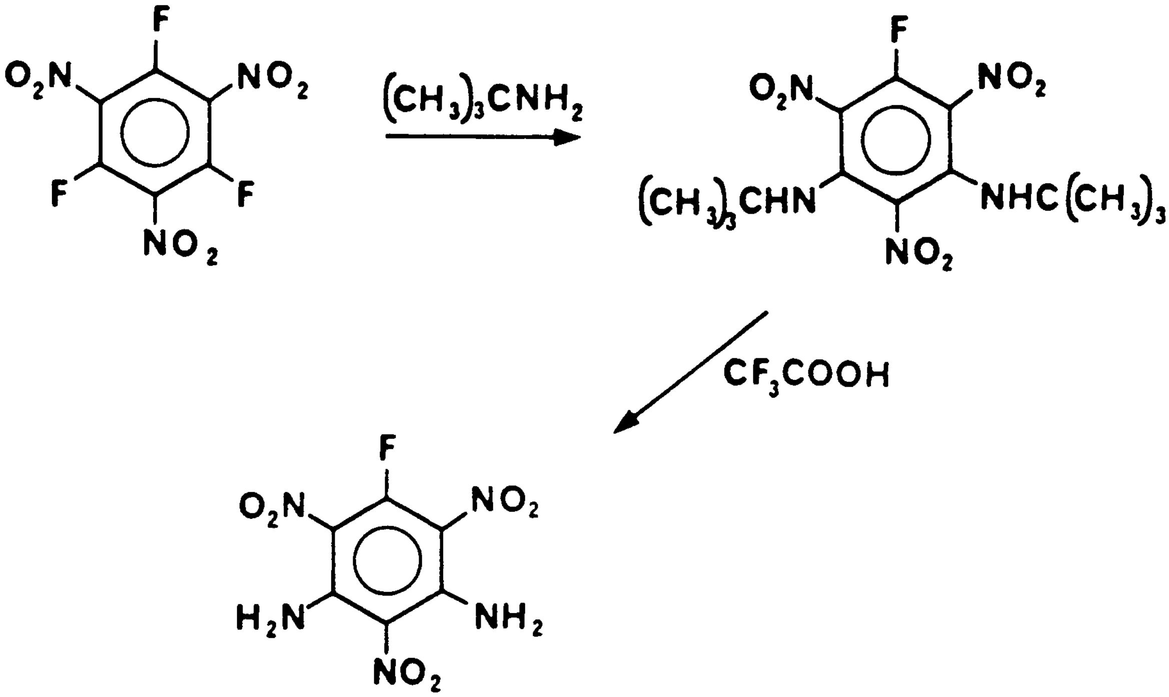


Les impuretés trisubstituées (TATB) et monosubstituées peuvent être aisément éliminées respectivement par filtration et par recristallisation.

Cette synthèse a été décrite en particulier par W.M. Koppes dans le brevet américain US-A-4 173 591.

Le 3,5-diamino-1-chloro-2,4,6-trinitrobenzène peut être préparé d'une manière analogue en partant du trichlorotrinitrobenzène beaucoup plus accessible commercialement.

Le 3-nitro-1,2,4-triazole est un produit du commerce. Il peut aussi être préparé à partir du 3-amino-1,2,4-triazole par des méthodes classiques. Une méthode couramment utilisée consiste à effectuer une diazotation par action de l'acide nitreux puis une substitution du groupe diazonium par l'ion nitrite $NO_2^-$.

Comme on l'a indiqué précédemment la réaction du 3,5-diamino-1-fluoro-2,4,6-trinitrobenzène avec le 3-nitro-1,2,4-triazole peut être effectuée facilement à la température ambiante.

On opère avantageusement dans un milieu réactionnel exempt d'eau, en utilisant un solvant organique tel que le diméthylformamide. Les produits de départ doivent être séchés préalablement sous vide et le solvant organique doit être déshydraté sur un tamis moléculaire, puis distillé. On effectue de préférence la réaction sous un balayage de gaz neutre tel que l'argon ou l'azote parfaitement déshydraté.

Dans le cas où l'halogénure utilisé est le 3,5-diamino-1-chloro-2,4,6-trinitrobenzène, on forme préalablement un sel alcalin du 3-nitro-1,2,4-triazole pour faciliter la réaction car la réactivité de l'atome de chlore est trop faible pour que la substitution nucléophile puisse être faite directement. Dans ce cas, on fait réagir le 3-nitro-1,2,4-triazole avec un alcoolate de métal alcalin ROM avec M représentant un métal alcalin tel que Li, Na ou K.

L'alcoolate peut être obtenu par la réaction suivante :

$$R\text{-}OH + M \rightarrow R - OM + 1/2H_2$$

avec R représentant un radical alkyle de 1 à 3 atomes de carbone.

Dans ce cas, la synthèse correspond au schéma réactionnel suivant :

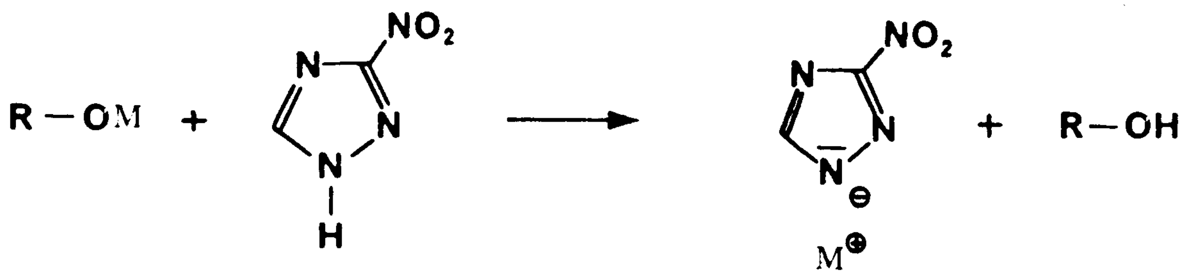

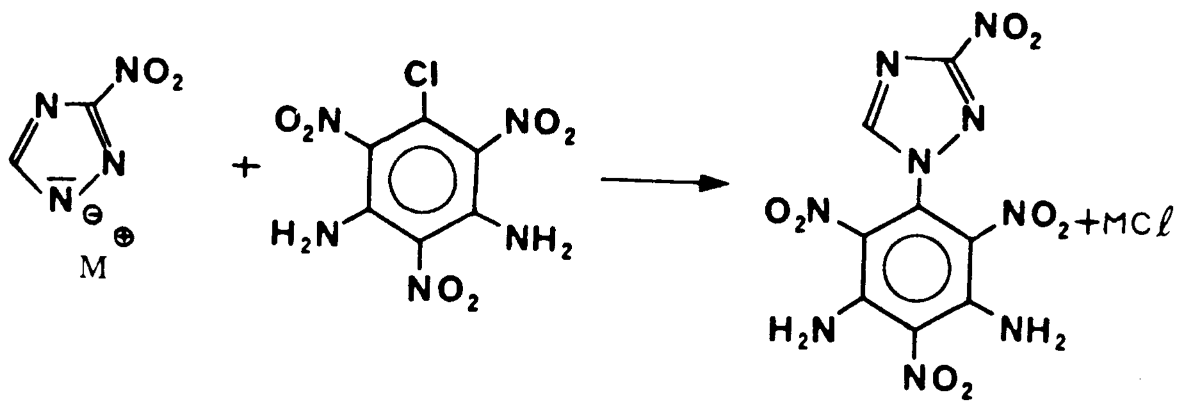

avec M = Li, Na, K.

De préférence, on opère à une température supérieure à la température ambiante, par exemple vers 50 ° C.

Le dérivé de triazole de l'invention peut être utilisé comme matériau explosif. Dans ce cas, le dérivé de triazole est généralement dispersé dans un liant thermoplastique ou thermodurcissable, contenant éventuellement d'autres additifs habituellement utilisés dans de telles compositions (plastifiants, etc).

D'autres caractéristiques et avantages de l'invention apparaîtront mieux à la lecture des exemples suivants donnés bien entendu à titre illustratif et non limitatif.

Exemple 1 : Préparation du 5-nitro-2(3,5-diamino-2,4,6-trinitrophényl)-1,2,4-triazole à partir du 1-fluoro-3,5-diamino-2,4,6-trinitrobenzène.

I. Synthèse du 1-fluoro-3,5-diamino-2,4,6-trinitrobènzene.

On réalise la synthèse de ce dérivé fluoré par réaction du 1,3,5-trifluoro-2,4,6-trinitrobenzène avec la tert-butylamine.

a) Préparation du 1,3,5-trifluoro-2,4,6-trinitrobenzène.

On réalise cette préparation en utilisant le mode de synthèse décrit dans le brevet américain US-A-4 173 591, en opérant de la façon suivante.

On ajoute peu à peu 48g de nitrate de potassium finement broyé à une solution de 200 ml d'acide sulfurique fumant à 30%, tout en refroidissant pour ne pas dépasser une température de 50 ° C. On ajoute ensuite 10g de 1,3,5-trifluorobenzène et on porte la solution à 156 ° C pendant 72 heures. Après refroidissement à la température ambiante, on extrait la solution avec 3 fois 250 ml de $CH_2Cl_2$. On sèche ensuite les phases organiques sur $Na_2SO_4$. On concentre la phase organique et on ajoute de l'hexane. On obtient ainsi un précipité de 1,3,5-trifluoro-2,4,6-trinitrobenzène, avec un rendement de nitration de 40 à 50%.

b) Préparation du 1-fluoro-3,5-diamino-2,4,6-trinitrobenzène.

Dans un réacteur de 2l muni d'un système d'agitation, d'un thermomètre, d'un système réfrigérant et d'une arrivée d'azote, on introduit 5g de trifluorotrinitrobenzène obtenu à l'étape a) dans 200 ml de $CH_2Cl_2$ séché sur chlorure de calcium, et 7,5g de bicarbonate de sodium.

On porte le milieu réactionnel à -30 ° C et on y introduit, à cette température, 2,75g de tert-butylamine (séchée sur potasse et distillée) dans 750 ml de $CH_2Cl_2$ sec, en réalisant l'introduction sur une durée de 2h30.

A la fin de cette opération, on laisse le milieu réactionnel revenir à la température ambiante tout en maintenant l'agitation sous azote pendant 15h. On filtre alors le milieu réactionnel, et on évapore le solvant pour obtenir un produit brut contenant 3 composés. On hydrolyse alors le produit brut dans un mélange d'acide trifluorocétique et de dichlorométhane contenant 50 ml d'acide trifluoracétique et 10 ml de dichlorométhane, à la température ambiante pendant 20 heures. On filtre alors le milieu réactionnel et on extrait le produit avec 80 ml de 1,2-dichloréthane au reflux. Après concentration du filtrat à environ 75 ml, on purifie le produit par cristallisations successives. On obtient ainsi le 1-fluoro-3,5-diamino-2,4,6-trinitrobenzène avec un rendement d'environ 35%. Son point de fusion est de 222 ° C.

II. Préparation du 3-nitro-1,2,4-triazole.

On ajoute une solution de 16,8g (0,2 mole) de 3-amino-1,2,4-triazole dans 160 ml d'acide acétique glacial à une solution de 16g (0,23 mole) de nitrite de sodium dans 70 ml d'acide sulfurique concentré, à une température de 0 à -5 ° C. Après 5 min, on ajoute goutte à goutte 50 ml d'eau à une température n'excédant pas 0 ° C. On ajoute alors la solution obtenue à 200 ml de nitrite de sodium à 10%, à une température de 45 à 50 ° C. On chauffe ensuite à 45 ° C pendant 1h, puis on acidifie la solution avec 6 ml de $H_2SO_4$ pour faire disparaître les oxydes d'azote, et on la traite avec 12g d'urée afin de détruire les oxydes d'azote en solution. On extrait ensuite la solution avec de l'acétate d'éthyle.

Après élimination de l'acétate d'éthyle par évaporation, on recristallise le produit dans du méthanol et on obtient ainsi 13g de 3-nitro-1,2,4-triazole. Son point de fusion est de 210 ° C. Le rendement est de 57%.

III. Synthèse du 5-nitro-2(3,5-diamino-2,4,6-trinitrophényl)-1,2,4-triazole.

On dissout 2,2g du 3-nitro-1,2,4-triazole (0,0193 mole) obtenu à l'étape précédente II dans 150 ml de diméthylformamide (DMF), séchée sur tamis et distillée. On agite ensuite la solution à la température ambiante et sous balayage d'azote pendant 30 min.

On ajoute alors 5g (0,192 mole) de 1-fluoro-3,5-diamino-2,4,6-trinitrobenzène et on maintient la solution sous agitation pendant 24 heures. On verse ensuite la solution dans un litre d'eau froide sous agitation.

On filtre le produit brut, on le lave plusieurs fois à l'eau puis à l'éther et on le sèche. On obtient ainsi 6,6g de 5-nitro-2(3,5-diamino-2,4,6-trinitrophényle)-1,2,4-triazole. Le rendement est de 95%.

L'analyse élémentaire du produit donne les résultats suivants :

| Analyse élémentaire (produit brut) : | | | |
|---|---|---|---|
| | C | H | N |
| Trouvée<br>Calculée | 26,95<br>27,05 | 1,41<br>1,42 | 35,53<br>35,49 |

Le produit se présente sous la forme de cristaux jaunes. Il présente une densité élevée : la densité de cristal mesurée par la méthode de flottation est de 1,93. Il se décompose sans fusion préalable à partir de 260 ° C. Il est insoluble dans l'eau et dans la plupart des solvants organiques usuels. Il est soluble dans le diméthylformamide (DMF) et le diméthylsulfoxyde (DMSO).

Il présente les propriétés suivantes :

1) Propriétés spectroscopiques

a) Résonance magnétique nucléaire (RMN)

En adoptant pour les différents noyaux de la molécule les notations suivantes :

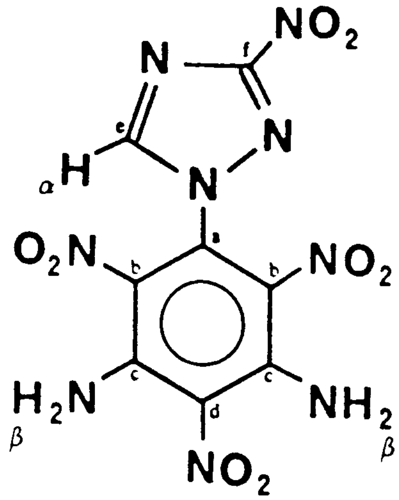

les déplacements chimiques par rapport au tétraméthylsilane (TMS) obtenus par spectroscopie de résonance magnétique nucléaire sont les suivants :

- En RMN du carbone $^{13}C$ à 20,15 MHz, l'échantilllon étant en solution dans la diméthylformamide deutériée.

| | |
|---|---|
| $C_f$ : 162,9 ppm | $C_B$ : 122,8 ppm |
| $C_e$ : 149,2 ppm | $C_c$ : 143,3 ppm |
| $C_a$ : 130,2 ppm | $C_d$ : 121,7 ppm. |

- En RMN $^1H$ à 60 MHz, l'échantillon étant en solution dans le diméthylsulfoxyde (DMSO).

$H\alpha$ : 9,34 ppm
$H\beta$ : 8,30 ppm.

b) Spectroscopie infrarouge

L'analyse par spectroscopie infrarouge du 5-nitro-2(3,5-diamino-2,4,6-trinitrophényl)-1,2,4-triazole, sous forme de dispersion dans des pastilles de KBr, donne les bandes caractéristiques suivantes :
$NH_2$ à 3422 $cm^{-1}$ ($\nu_{as}$) ; 3313 $cm^{-1}$ ($\nu_s$) et 1616 $cm^{-1}$ (déformation) $NO_2$ à 1573 $cm^{-1}$ ($\nu_{as}$) ; 1301 et 1281 $cm^{-1}$ ($\nu_s$)
CH du cycle triazole à 3141 $cm^{-1}$.

2) Propriétés détoniques

a) Balance en oxygène

La balance en oxygène par rapport au $CO_2$ et $H_2O$ est de -47,30g d'$O_2$ pour 100g d'explosif.

Le défaut en oxygène est donc moins important que dans le cas du triaminotrinitrobenzène TATB (-55,81g/100g) ou de l'hexanitrostilbène (-67,5 g/100g) de formule :

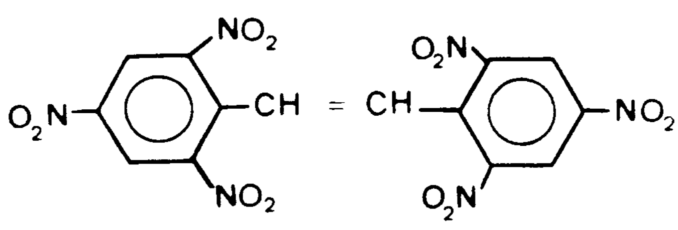

Il est plus important que dans le cas de l'octogène (-21,6 g/100g).

b) Température de déflagration

Un échantillon du produit (20 mg) en conteneur d'acier inoxydable est trempé dans un bain dont la température est élevée à 5 ° C/min. Dans le cas présent, la température à laquelle le produit déflagre est de 260 ° C.

c) Temps d'induction thermique

10 mg du produit dans un conteneur d'acier sont introduits brusquement dans un bain à la température de mesure. On enregistre le temps au bout duquel on a décomposition. En considérant que la cinétique est d'ordre zéro, on peut calculer l'énergie d'activation.

Pour le 5-nitro-2(3,5-diamino-2,4,6-trinitrophényl)-1,2,4-triazole, la déflagration se produit au bout de 5 secondes pour une température de 288 ° C.

L'énergie d'activation est de 23,9 kcal/mole.

d) Vitesse de détonation

La vitesse de détonation calculée à partir de la formule du composé selon la méthode de Rothstein et Petersen décrite dans Propellants and Explosives 4, 56-60 (1979) est, pour la densité de cristal, de 8240 m/s.

A titre de comparaison, selon cette même méthode la vitesse de détonation du TATB est de 7870 m/s et celle de l'octogène est de 9050 m/s.

e) Sensibilité au choc

La sensibilité au choc du 5-nitro-2(3,5-diamino-2,4,6-trinitrophényl)-1,2,4-triazole a été déterminée à l'aide d'un mouton pendulaire de 5 kg, l'échantillon de 30 mg étant déposé sur papier de verre. La conduite de l'essai se fait selon la méthode de Bruceton.

La hauteur H et l'énergie $E_j$ correspondante, entraînant une probabilité de réaction pyrotechnique de 0,5 sont :

$H_{(50)}$ = 51,3 cm

$E_j$ = 25,2 J.

Le 5-nitro-2(3,5-diamino-2,4,6-trinitrophényl)-1,2,4-triazole apparaît donc comme un explosif de faible sensibilité au choc, intermédiaire entre l'octogène ($H_{(50)}$ = 15 cm), et le TATB ($H_{(50)}$ >72 cm).

Example 2 : Préparation du 5-nitro-2(3,5-diamino-2,4,6-trinitrophényl)-1,2,4-triazole à partir du 1-chloro-3,5-diamino-2,4,6-trinitrobenzène.

I. Préparation du 1-chloro-3,5-diamino-2,4,6-trinitrobenzène.

Dans un réacteur de 4 litres équipé d'un agitateur, d'un réfrigérant et d'un thermomètre, on place :
- 1,5 litre de dichlorométhane séché sur $CaCl_2$,
- 22 g (0,3 mole) de tert-butylamine distillée,
- 12,6 g de bicarbonate de sodium finement broyé.

On ajoute alors, goutte à goutte, une solution de 11,7g (0,037 mole) de trichlorotrinitrobenzène (recristallisé trois fois dans le chlorobenzène) dans 400 ml de $CH_2Cl_2$ sec sur un temps d'environ 2 heures. On porte ensuite le mélange réactionnel au reflux pendant 15 heures.

Après évaporation du dichlorométhane, on obtient un produit brut que l'on hydrolyse pendant 20 heures à la température ambiante à l'aide d'un mélange acide trifluoroacétique/dichlorométhane (50 ml/10 ml).

On récupère le 1-chloro-3,5-diamino-2,4,6-trinitrobenzène par extraction au 1,2 dichloroéthane au reflux, puis cristallisations successives. Le rendement est de 10 à 12%.

L'analyse élémentaire du produit est la suivante :

| | C | H | N |
|---|---|---|---|
| Trouvée | 26,06 | 1,41 | 24,96 |
| Calculée | 25,96 | 1,45 | 25,23 |

II. Préparation du 5-nitro-2(3,5-diamino-2,4,6-trinitrophényl)-1,2,4-triazole.

Dans un mélange de 50 ml d'éthanol absolu et de 120 ml de n-hexane (séché sur sodium), on ajoute 0,8 g de sodium (0,0348 mole). Après réaction, on introduit 3,9g de 3-nitro-1,2,4-triazole (0,0342 mole) obtenu dans l'exemple 1 (étape II) et on porte au reflux pendant 30 minutes. On refroidit vers 30 ° C puis on ajoute 9,5g de 1-chloro-3,5-diamino-2,4,6-trinitrobenzène (0,0342 mole) et on maintient 2 heures sous agitation à 50 ° C. On filtre le produit brut puis ou le rince à l'éther froid.

On obtient ainsi 13,5g de produit brut 5-nitro-2(3,5-diamino-2,4,6-trinitrophényl)-1,2,4-triazole. Pour chasser le chlorure de sodium formé, on dissout le produit brut dans du DMF à la température ambiante puis on filtre pour séparer NaCl. On verse ensuite la solution dans 600 ml d'eau froide. On filtre le produit, on le lave à l'éther et on le sèche. On obtient 8,5g. Le rendement est d'environ 70%.

L'analyse élémentaire du produit donne les résultats suivants :

| | C | H | N |
|---|---|---|---|
| Trouvée | 27,02 | 1,33 | 35,10 |
| Calculée | 27,05 | 1,42 | 35,49 |

Ce produit présente des propriétés physiques, spectroscopiques et détoniques, identiques à celles que l'on obtient avec le produit préparé selon l'exemple 1.

## Revendications

**1.** 5-nitro-2(3,5-diamino-2,4,6-trinitrophényl)-1,2,4-triazole, répondant à la formule :

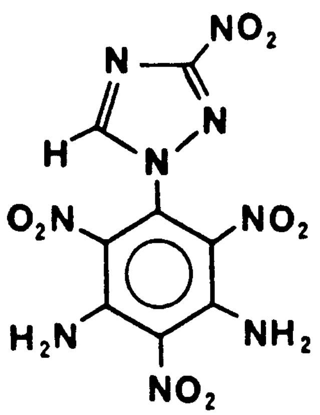

(I)

**2.** Procédé de préparation du 5-nitro-2(3,5-diamino-2,4,6-trinitrophényl)-1,2,4-triazole de formule :

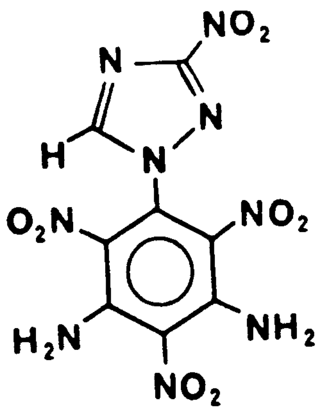

(I)

caractérisé en ce qu'il consiste à faire réagir un 3,5-diamino-1-halogéno-2,4,6-trinitrobenzène de formule :

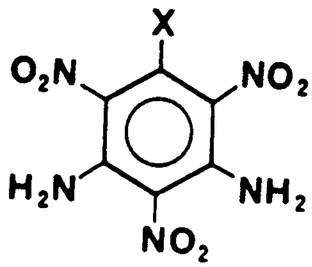

(II)

dans laquelle X représente un atome de chlore ou de fluor, avec un 3-nitro-1,2,4-triazole de formule :

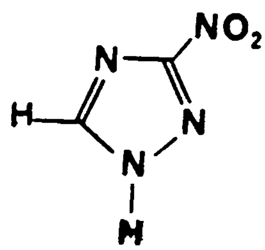

(III)

dans laquelle M représente un atome d'hydrogène ou de métal alcalin.

3. Procédé selon la revendication 2, caractérisé en ce que X représente un atome de fluor et M est un atome d'hydrogène.

4. Procédé selon la revendication 3, caractérisé en ce que la réaction est effectuée à la température ambiante dans un milieu organique exempt d'eau.

5. Procédé selon la revendication 2, caractérisé en ce que X représente un atome de chlore et M représente un atome de métal alcalin.

6. Procédé selon la revendication 5, caractérisé en ce que l'on forme tout d'abord un sel alcalin du 3-nitro-1,2,4-triazole par réaction du 3-nitro-1,2,4-triazole avec un alcoolate de métal alcalin.

7. Procédé selon l'une quelconque des revendications 5 et 6, caractérisé en ce que le métal alcalin est choisi parmi le lithium, le sodium et le potassium.

8. Matériau explosif caractérisé en ce qu'il comprend du 5-nitro-2(3,5-diamino-2,4,6-trinitrophényl)-1,2,4-triazole de formule :

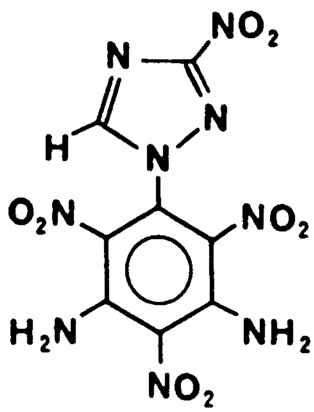

(I)

## Claims

**1.** 5-nitro-2(3,5-diamino-2,4,6-trinitrophenyl)-1,2,4-triazole in accordance with formula:

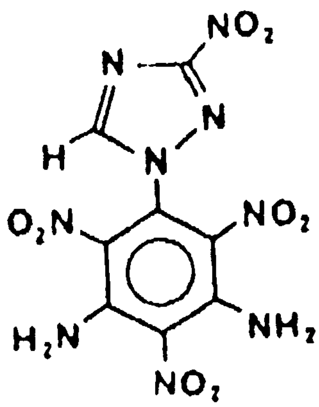

(I)

**2.** Process for the preparation of 5-nitro-2(3,5-diamino-2,4,6-trinitrophenyl)-1,2,4-triazole of formula:

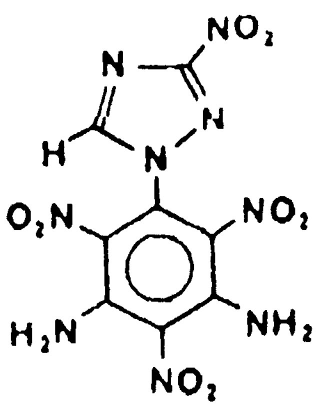

(I)

characterized in that it comprises reacting a 3,5-diamino-1-halogeno-2,4,6-trinitrobenzene of formula:

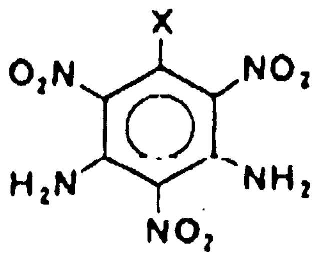

(II)

in which X represents a chlorine or fluorine atom with a 3-nitro-1,2,4-triazoleof formula:

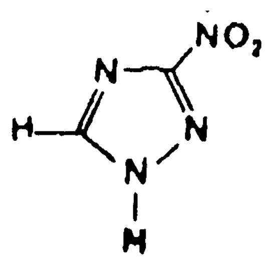

(III)

in which M represents an alkali metal or hydrogen atom.

3. Process according to claim 2, characterized in that X represents a fluorine atom and M a hydrogen atom.

4. Process according to claim 3, characterized in that the reaction is performed at ambient temperature in a water-free organic medium.

5. Process according to claim 2, characterized in that X represents a chlorine atom and M an alkali metal atom.

6. Process according to claim 5, characterized in that firstly an alkali metal salt of 3-nitro-1,2,4-triazole is formed by reacting 3-nitro-1,2,4-triazole with an alkali metal alkoxide.

7. Process according to claim 2, characterized in that the alkali metal is chosen from among lithium, sodium and potassium.

8. Explosive material, characterized in that it comprises 5-nitro-2(3,5-diamino-2,4,6-trinitrophenyl)-1,2,4-triazole of formula:

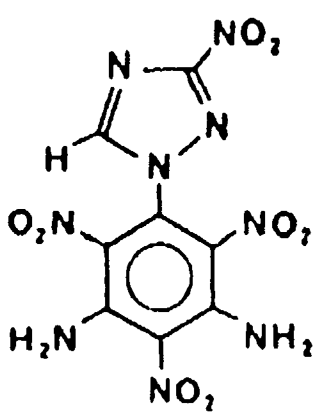

(I)

**Patentansprüche**

1. 5-Nitro-2-(3,5-diamino-2,4,6-trinitrophenyl)-1,2,4-triazol entsprechend der Formel

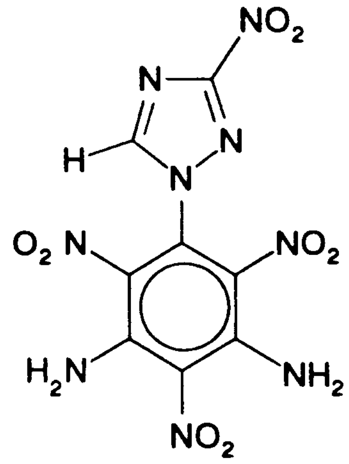

(I)

2. Verfahren zur Herstellung von 5-Nitro-2-(3,5-diamino-2,4,6-trinitrophenyl)-1,2,4-triazol der Formel

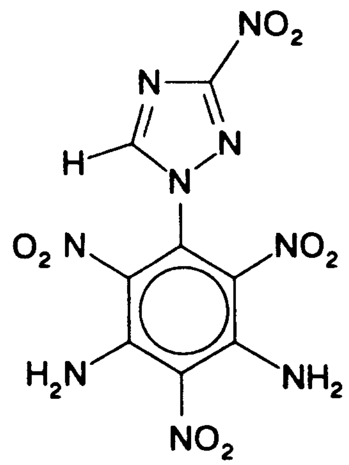

(I)

dadurch gekennzeichnet, daß man ein 3,5-Diamino-1-halogen-2,4,6-trinitrobenzol der Formel

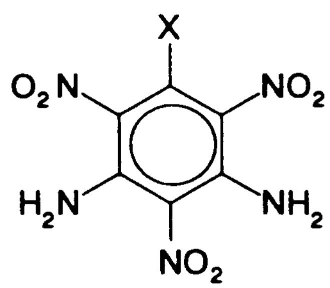

(II)

worin X ein Chlor- oder Fluor-Atom bedeutet, mit einem 3-Nitro-1,2,4-triazol der Formel

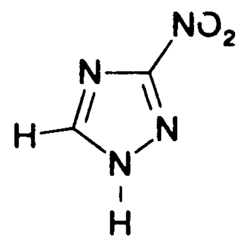

(III)

worin M ein Wasserstoff-Atom oder ein Alkalimetall bedeutet, umsetzt.

**3.** Verfahren gemäß Anspruch 2, dadurch gekennzeichnet, daß X ein Fluor-Atom bedeutet und M ein Wasserstoff-Atom ist.

**4.** Verfahren gemäß Anspruch 3, dadurch gekennzeichnet, daß die Reaktion bei Raumtemperatur in einem organischen Milieu außer Wasser durchgeführt wird.

**5.** Verfahren gemäß Anspruch 2, dadurch gekennzeichnet, daß X ein Chlor-Atom bedeutet und M ein Alkalimetall-Atom ist.

**6.** Verfahren gemäß Anspruch 5, dadurch gekennzeichnet, daß man zuerst ein Alkalisalz des 3-Nitro-1,2,4-triazols durch Umsetzung des 3-Nitro-1,2,4-triazols mit einem Alkalimetallalkoholat bildet.

**7.** Verfahren gemäß einem der Ansprüche 5 und 6, dadurch gekennzeichnet, daß das Alkalimetall aus Lithium, Natrium und Kalium ausgewählt ist.

**8.** Explosivstoff, dadurch gekennzeichnet, daß er 5-Nitro-2-(3,5-diamino-2,4,6-trinitrophenyl)-1,2,4-triazol entsprechend der Formel

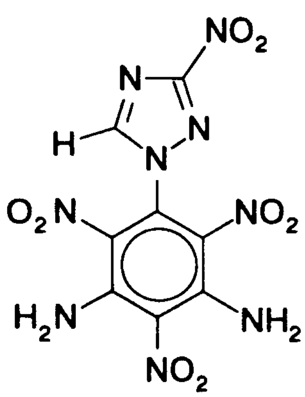

(I)

enthält.